# EUROPEAN PATENT APPLICATION

(11) **EP 1 683 806 A1**
(43) Date of publication of application: **26.07.2006**
(21) Application number: 06001037.8
(22) Date of filing: 18.01.2006
(51) Int. Cl.: C07H 19/173

(54) **Production method of 2'-deoxyguanosine compound**

(30) Priority: 21.01.2005 JP 2005014804
(71) Applicant: Ajinomoto Co., Inc., Chuo-ku, Tokyo (JP)
(72) Inventor: Nishikawa, Toshihiro, Kawasaki-shi Kanagawa 210-8681 (JP); Onishi, Tomoyuki, Kawasaki-shi Kanagawa 210-8681 (JP)
(74) Representative: Strehl Schübel-Hopf & Partner

(57) **Abstract**

The present invention provides a production method of a 2'-deoxyguanosine compound represented by the following formula (2), which includes a step of desulfurization reaction of an 8,2'-anhydro-8-mercapto-9-β-arabinofuranosylguanine compound represented by the following formula (1) in a solvent in the presence of a base using nickel or a nickel alloy. 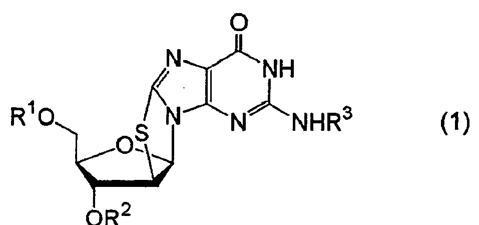 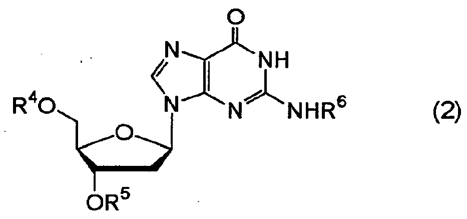 wherein R¹, R², R⁴ and R⁵ are each independently a hydrogen atom or a hydroxyl-protecting group, and R³ and R⁶ are hydrogen atoms or amino-protecting groups.

## Description

### TECHNICAL FIELD OF THE INVENTION

The present invention relates to a production method of a 2'-deoxyguanosine compound. More particularly, the present invention relates to a production method of a 2'-deoxyguanosine compound useful as a starting material for the production of an antisense drug.

### BACKGROUND OF THE INVENTION

All biological organisms have a gene, a biopolymer, and the gene transmits genetic information to the living organisms by being transcribed to mRNA and then translated into a protein. In the case of a disease caused by a genetic factor, it is considered that the disease can be treated by controlling expression of the protein by blocking the flow of genetic information of the pathogenic gene. In recent years, an antisense therapy that base sequence-specifically suppresses expression of such pathogenic gene has been drawing attention. The antisense drug used for such therapy consists of oligonucleotide and, as a starting material for the production of oligonucleotide, deoxynucleoside is known (Follmann H. Chem. Soc. Rev. 2004, 33, 225-233).

As a production method of deoxynucleoside, for example, the following method using an 8,2'-anhydro-8-mercapto-9-β-arabinofuranosylpurine derivative as a starting material has been reported. Ikehara M. et al., Chem. Pharm. Bull. 1966, 15(1), 94-100, disclose production methods of 2'-deoxyadenosine, in which 8,2'-anhydro-8-mercapto-9-β-arabinofuranosyladenine is desulfurized in the presence of about 700 mass% of Raney-nickel in an aqueous solution to give 2'-deoxyadenosine in a yield of 33-40%. In addition, Yamazaki A. et al., Chem. Pharm. Bull. 1973, 21(5), 1143-1146 discloses a production method of 2'-deoxyinosine, in which 8,2'-anhydro-8-mercapto-9-β-arabinofuranosylhypoxanthine is desulfurized in the presence of 6.7 ml of Raney-nickel per 1 g of 8,2'-anhydro-8-mercapto-9-β-arabinofuranosylhypoxanthine, a starting material, in an aqueous solution to give 2'-deoxyinosine in a yield of 60%. Moreover, Ogilvie K.K. et al., Can. J. Chem., 1972, 50, 1100-1104, and Kaneko M. et al., Chem. Pharm. Bull. 1972, 20(3), 635-637 disclose production methods of 2'-deoxyguanosine, in which 8,2'-anhydro-8-mercapto-9-β-arabinofuranosylguanine was desulfurized in the presence of Raney-nickel in an aqueous solution merely to confirm the presence of 2'-deoxyguanosine by thin layer chromatography (TLC), without description of the amount of Raney-nickel used and yield.

### SUMMARY OF THE INVENTION

The present inventors considered production of 2'-deoxyguanosine by reference to the descriptions of the above-mentioned references, only to find the problem that the desulfurization reaction of 8,2'-anhydro-8-mercapto-9-β-arabinofuranosylguanine with Raney-nickel hardly proceeds.

It is therefore an object of the present invention to provide an economical and efficient production method of a 2'-deoxyguanosine compound, which is suitable for industrial production.

As a result of the intensive studies in an attempt to solve the above-mentioned problems, the present inventors have found that one of the causes of the insufficient yield by conventional production methods of 2'-deoxyguanosine is the solubility of 8,2'-anhydro-8-mercapto-9-β-arabinofuranosylguanine in the reaction system. They have further studied based on the finding and found that a 2'-deoxyguanosine compound can be synthesized in a high yield by desulfurization reaction of an 8,2'-anhydro-8-mercapto-9-β-arabinofuranosylguanine compound in the presence of nickel or a nickel alloy, a solvent, and a base. Particularly, by the use of a nickel-aluminum alloy for the reaction, the amount of a nickel reagent to be used can be markedly reduced as compared to the use of Raney-nickel, based on which finding the present invention has been completed.

Accordingly, the present invention is characterized by the following.
[1] A method of producing a 2'-deoxyguanosine compound represented by the following formula (2) 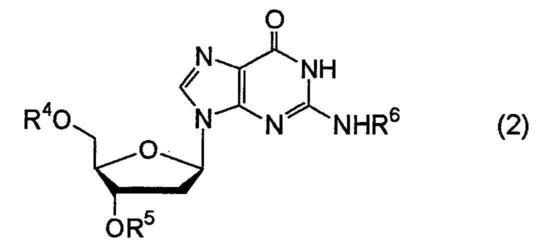 wherein R⁴ and R⁵ are each independently a hydrogen atom or a hydroxyl-protecting group and R⁶ is a hydrogen atom or an amino-protecting group, which comprises desulfurizing an 8,2'-anhydro-8-mercapto-9-β-arabinofuranosylguanine compound represented by the following formula (1) 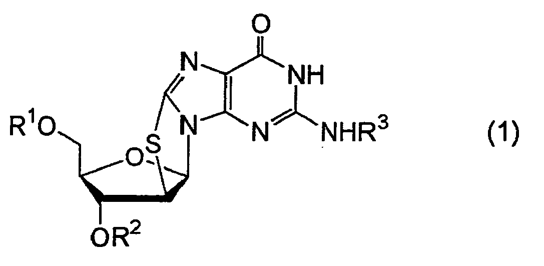 wherein R¹ and R² are each independently a hydrogen atom or a hydroxyl-protecting group and R³ is a hydrogen atom or an amino-protecting group, in the presence of nickel or a nickel alloy, a solvent, and a base.
[2] A method of producing a 2'-deoxyguanosine compound represented by the following formula (3) 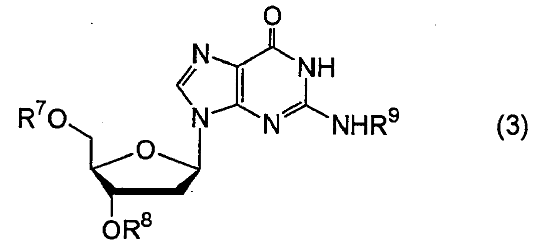 wherein R⁷ and R⁸ are each independently a hydrogen atom or a hydroxyl-protecting group, R⁹ is a hydrogen atom or an amino-protecting group, provided that R⁷, R⁸ and R⁹ are not simultaneously hydrogen atoms, which comprises obtaining a 2'-deoxyguanosine compound represented by the formula (2) according to the production method of the above-mentioned [1], and when one or more of R⁴, R⁵ and R⁶ of the compound are hydrogen atoms, replacing one or more of the hydrogen atoms with protecting group(s).
[3] The production method of the above-mentioned [1], wherein R¹, R², R³, R⁴, R⁵ and R⁶ are hydrogen atoms.
[4] The production method of the above-mentioned [3], which further comprises a step of replacing hydrogen atoms for R⁴, R⁵ and R⁶ with protecting groups.
[5] The production method of any of the above-mentioned [1]-[4], wherein the solvent is water.
[6] The production method of any of the above-mentioned [1]-[5], wherein the desulfurization is carried out in a liquid which comprises a base and a solvent and which has a pH of not less than 11.
[7] The production method of any of the above-mentioned [1]-[6], wherein the nickel or nickel alloy is added in an amount of 50-1500 mass% of the total mass of the 8,2'-anhydro-8-mercapto-9-β-arabinofuranosylguanine compound.
[8] The production method of any of the above-mentioned [1]-[7], wherein the desulfurization reaction is carried out at a temperature of 40-100°C.
[9] The production method of any of the above-mentioned [1]-[8], wherein the nickel alloy is a nickel-aluminum alloy.

### DETAILED DESCRIPTION OF THE INVENTION

The production method of the 2'-deoxyguanosine compound of the present invention is described in the following.

The production method of the 2'-deoxyguanosine compound of the present invention comprises, as shown in the following reaction scheme, desulfurizing an 8,2'-anhydro-8-mercapto-9-β-arabinofuranosylguanine compound represented by the following formula (1) in a solvent, in the presence of a base, using a nickel or nickel alloy to give a 2'-deoxyguanosine compound represented by the following formula (2):

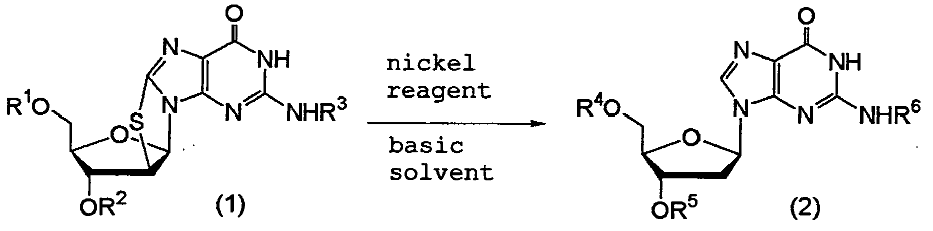

In the above-mentioned production method, when a nickel-aluminum alloy (Ni-Al alloy) is used as the nickel reagent, the Ni-Al alloy is developed to Raney-nickel in a solvent in the presence of a base during a desulfurization reaction. Then, an 8,2'-anhydro-8-mercapto-9-β-arabinofuranosylguanine compound is desulfurized by the reducibility of the Raney-nickel to give a 2'-deoxyguanosine compound. In this case, the amount of the nickel reagent to be used can be markedly reduced and the production cost can be decreased. Moreover, since preparation of Raney-nickel before desulfurization reaction becomes unnecessary, a 2'-deoxyguanosine compound can be safely produced. The present inventors know that, when the desulfurization reaction is carried out using Raney-nickel prepared before desulfurization reaction in an aqueous solution in the presence of a base, Raney-nickel needs to be added in an amount of not less 1500 mass% per 8,2'-anhydro-8-mercapto-9-β-arabinofuranosylguanine compound to achieve the same level of yield as does Ni-Al alloy.

In the formulas of the present invention, R¹, R², R⁴ and R⁵ are each independently a hydrogen atom or a hydroxyl-protecting group. The hydroxyl-protecting group is not particularly limited and the substituents described in Protecting Groups in Organic Synthesis 3rd edition (Wiley-Interscience, Inc. 1999) can be used. Specifically, for example, acyl group, alkyl group, aralkyl group, silyl group and the like can be mentioned. As the acyl group, for example, acyl group having 1 to 7 carbon atoms, such as formyl group, acetyl group, isobutyryl group, pivaloyl group, benzoyl group and the like can be mentioned. As the alkyl group, for example, alkyl group having 1 to 7 carbon atoms, such as methyl group, ethyl group, n-propyl group, isopropyl group, n-butyl group, tert-butyl group and the like can be mentioned. As the aralkyl group, for example, aralkyl group having 7 to 22 carbon atoms, such as benzyl group, 4-monomethoxybenzyl group, trityl group, 4-monomethoxytrityl group, 4,4'-dimethoxytrityl group and the like can be mentioned. As the silyl group, for example, tri-substituted silyl group such as trimethylsilyl group, triethylsilyl group, tert-butyldimethylsilyl group and the like can be mentioned. In addition, hydroxyl-protecting group such as methoxymethyl group, methylthiomethyl group, benzyloxymethyl group, methoxyethoxymethyl group, tetrahydropyranyl group, methoxycarbonyl group, 9-fluorenylmethoxycarbonyl group, 2,2,2-trichloroethoxycarbonyl group, benzyloxycarbonyl group, tert-butoxycarbonyl group and the like can be used. Since reaction is carried out under basic conditions, trityl group, 4-monomethoxytrityl group, 4,4'-dimethoxytrityl group and a hydrogen atom are preferable, and a hydrogen atom is particularly preferable as R¹, R², R⁴ or R⁵.

In the formulas of the present invention, R³ and R⁶ is each a hydrogen atom or an amino-protecting group. The amino-protecting group is not particularly limited and the substituents described in Protecting Groups in Organic Synthesis 3rd edition (Wiley-Interscience, Inc. 1999) can be used. Specifically, alkyl group, aralkyl group, acyl group, alkoxycarbonyl group, alkylidene group and the like can be mentioned. As the alkyl group, for example, alkyl group having 1 to 10 carbon atoms such as methyl group, ethyl group, n-propyl group, isopropyl group, n-butyl group, tert-butyl group and the like can be mentioned. As the aralkyl group, for example, aralkyl group having 7 to 22 carbon atoms such as benzyl group, 4-monomethoxybenzyl group, trityl group, 4-monomethoxytrityl group, 4,4'-dimethoxytrityl group and the like can be mentioned. As the acyl group, for example, acyl group having 1 to 10 carbon atoms such as formyl group, acetyl group, isobutyryl group, pivaloyl group, benzoyl group, phenacyl group and the like can be mentioned. As the alkoxycarbonyl group, benzyloxycarbonyl group, tert-butoxycarbonyl group, methoxycarbonyl group and the like can be mentioned. As the alkylidene group, benzylidene group, diphenylmethylidene group, bis(methylthio)methylidene group, dimethylaminomethylidene group and the like can be mentioned. Since reaction is carried out under basic conditions, trityl group, 4-monomethoxytrityl group, 4,4'-dimethoxytrityl group and a hydrogen atom are preferable, and a hydrogen atom is particularly preferable as R³ or R⁶.

While the 8,2'-anhydro-8-mercapto-9-β-arabinofuranosylguanine compound has enantiomers, a D-form, an L-form or a mixture thereof may be used. An 8,2'-anhydro-8-mercapto-9-β-arabinofuranosylguanine compound can be produced according to the method described in Chem. Pharm. Bull. 1972, 20(3), 635-637. Specifically, guanosine (I) is first reacted with bromine to synthesize 8-bromoguanosine (II). Then, 8-bromoguanosine (II) is reacted with thiourea to synthesize 8-mercaptoguanosine (III), after which 8-mercaptoguanosine (III) is reacted with diphenyl carbonate to give 8,2'-anhydro-8-mercapto-9-β-arabinofuranosylguanine (IV). Protecting group(s) may be introduced into the hydroxyl group and amino group of 8,2'-anhydro-8-mercapto-9-β-arabinofuranosylguanine (IV) to give 8,2'-anhydro-8-mercapto-9-β-arabinofuranosylguanine compound (1). As the reagents for introducing protecting groups, any compounds can be used without any particular limitation as long as they are capable of affording protecting groups represented by R¹, R² and R³. For example, acid anhydride such as acetic anhydride etc., acid chloride such as acetyl chloride etc., and silane compound such as t-butyldimethylsilyl chloride etc. can be mentioned. To prevent release of the protecting group, acid anhydride is preferable and acetic anhydride is more preferable. 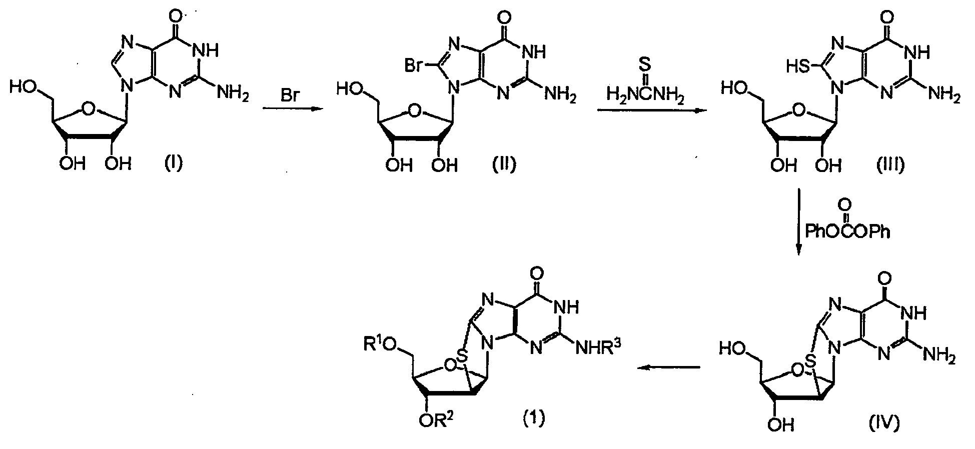 wherein R¹, R² and R³ are as defined above.

The 2'-deoxyguanosine compound represented by the formula (2) wherein one or more of R⁴, R⁵ and R⁶ of the compound are hydrogen atoms may be led to a 2'-deoxyguanosine compound represented by the following formula (3) by replacing one or more hydrogen atoms with protecting groups (protecting one or more of hydroxyl group and amino group with protecting groups).

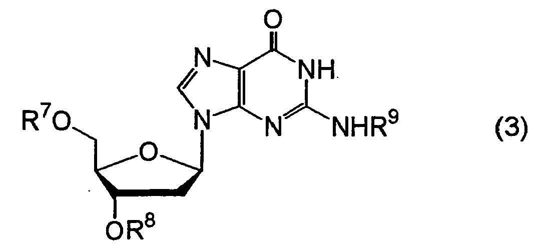

In the above-mentioned formula, R⁷ and R⁸ are each independently a hydrogen atom or a hydroxyl-protecting group and R⁹ is a hydrogen atom or an amino-protecting group, provided that R⁷, R⁸ and R⁹ are not simultaneously hydrogen atoms. As the hydroxyl-protecting group and amino-protecting group in this case, those similar to the ones mentioned above can be mentioned. Particularly, as the protecting groups for hydroxyl group and amino group, acyl group is preferable and isobutyryl group is more preferable.

As the nickel alloy, Ni-Al alloy is preferable. As the Ni-Al alloy, one having a nickel and aluminum mass ratio of Ni:Al of 50-40:50-60 is preferable to allow easy development under basic conditions. The Ni-Al alloy may contain other metal elements (e.g., iron, molybdenum) as necessary. As the nickel, Raney-nickel obtained by treating a nickel alloy such as Ni-Al alloy etc. with sodium hydroxide and washing same with water is preferably mentioned.

In the present invention, the desulfurization reaction is carried out in the presence of a base. As the base, sodium hydroxide, potassium hydroxide, lithium hydroxide, sodium carbonate, potassium carbonate, lithium carbonate, ammonia, ammonium hydroxide and the like can be mentioned, and sodium hydroxide, potassium hydroxide and lithium hydroxide are particularly preferable. The desulfurization reaction is carried out in a solvent in the present invention. As the solvent, water, methanol, ethanol, dimethyl sulfoxide, N,N-dimethylformamide, hexamethylphosphoric triamide, 2-methoxyethanol, ethylene glycol or a mixed solvent thereof can be used. As preferable solvent, water, methanol and ethanol can be mentioned, and water is particularly preferable. When the desulfurization reaction is carried out in an aqueous solvent, the pH of the aqueous solution before desulfurization reaction is preferably set to not less than 11, more preferably not less than 12, to efficiently produce Raney-nickel from Ni-Al alloy. It is desirable to prepare an aqueous solution to be used for the desulfurization reaction, for example, by adding a strong base such as sodium hydroxide, potassium hydroxide, lithium hydroxide etc. in water as a solvent to adjust the pH as mentioned above. In the production method of the present invention, it is preferable to dissolve an 8,2'-anhydro-8-mercapto-9-β-arabinofuranosylguanine compound in an aqueous solution and develop the Ni-Al alloy to Raney-nickel in the reaction system, under such conditions, thereby simultaneously carrying out the desulfurization reaction.

The desulfurization reaction can also be carried out under the ambient atmosphere. However, since nickel is easily oxidized by oxygen, the reaction is desirably carried out under an atmosphere of inert gas such as helium gas, argon gas, nitrogen gas and the like. Preferable reaction conditions of the desulfurization reaction are as follows. The reaction temperature is preferably 40-100°C, more preferably 60-100°C. When it is less than 40°C, the reactivity and reaction rate tend to decrease. The reaction time is preferably 1-60 hr, more preferably 1-20 hr. When it is less than 1 hr, the desulfurization reaction tends to be insufficient.

When nickel such as Raney-nickel etc. is used as the nickel reagent, the amount thereof to be used is preferably 50-1500 mass%, more preferably 500-1500 mass%, relative to the total mass of the 8,2'-anhydro-8-mercapto-9-β-arabinofuranosylguanine compound. When it is less than 500 mass%, the reactivity and reaction rate tend to decrease, and even when it is used in excess of 1500 mass%, an effect comparable to the amount of addition may not be obtained. When Ni-Al alloy is used, the amount thereof to be used is preferably 50-500 mass%, more preferably 150-500 mass%, relative to the total mass of the 8,2'-anhydro-8-mercapto-9-β-arabinofuranosylguanine compound. When it is less than 150 mass%, the reactivity and reaction rate tend to decrease, and even when it is used in excess of 500 mass%, an effect comparable to the amount of addition may not be obtained. The "mass%" is expressed relative to the mass of the 8,2'-anhydro-8-mercapto-9-β-arabinofuranosylguanine compound as the standard (100 mass%). The mass of the Raney-nickel is the mass of Ni-Al alloy as a starting material, namely, the mass of Ni-Al alloy (starting material of Raney-nickel) before development to Raney-nickel.

The obtained crude 2'-deoxyguanosine compound can be purified by a known method such as crystal precipitation etc. As mentioned above, 2'-deoxyguanosine is subjected to triacylation or di-tritylation without separation and purification, and then analysis by high performance liquid chromatography (HPLC), whereby purity and yield of the 2'-deoxyguanosine compound can be confirmed.
analysis conditions of high performance liquid chromatography (HPLC)
column: Inertsil ODS-AQ (GL Science Inc.), 4.6x250 nm, room temp.
mobile phase:
   A) 0.02M AcOH-AcONH₄ (pH 3.5)
   B) 0.02M AcOH-AcONH₄ (pH 3.5)/MeOH (60/40)
   8% (0-5 min), 8-100% (5-47 min)
flow rate: 0.7 mL/min
detector: UV 260 nm
injection volume: 10 µL (sample/0.01M aqueous sodium hydroxide solution)

The retention time of the 2'-deoxyguanosine compound is 17.5 min.

In another embodiment, the present invention provides a method for producing an antisense drug by converting a 2'-deoxyguanosine compound represented by either formula (2) or (3) into an oligonucleotide, in which the 2'-deoxyguanosine compound represented by either formula (2) or (3) is prepared according to the present method. The conversion of the 2'-deoxyguanosine compound into an oligonucleotide may be carried out by any suitable method (see, e.g., Follmann H., Chem. Soc. Rev., 2004, vol. 33, pp. 225-233, which is incorporated herein by reference in its entirety).

### EXAMPLES

The Examples of the present invention are explained in more detail in the following, which are not to be construed as limitative.

### (1) Production of 2'-deoxyguanosine

### (Example 1)

8,2'-Anhydro-8-mercapto-9-β-arabinofuranosylguanine (500 mg, 1.68 mmol), water (8.32 mL), 2M aqueous sodium hydroxide solution (1.68 mL) and Raney-nickel (7.5 g, Kawaken Fine Chemicals Co., Ltd., NDHT-M0) were added to a pear-shaped flask (50 mL) and the reaction mixture (pH 13.5) was stirred at 95°C for 15 hr. The reaction mixture was filtered through Kiriyama funnel under reduced pressure. The obtained solution was adjusted to pH 7 with 2M aqueous hydrochloric acid solution and the neutralized solution was concentrated under reduced pressure. The crystals precipitated during concentration were filtered through Kiriyama funnel under reduced pressure and the obtained crystals were vacuum dried for one day to give 2'-deoxyguanosine in a yield of 52% (0.87 mmol). The presence of 2'-deoxyguanosine (0.26 mmol) in the filtrate after filtration under reduced pressure was confirmed by high performance liquid chromatography (HPLC). The isolated 2'-deoxyguanosine and 2'-deoxyguanosine contained in the filtrate were obtained in a combined yield of 67%. ¹H-NMR (DMSO-d₆):δ 2.15-2.20 (m, 1H), 2.45-2.55 (m, 1H), 3.45-3.60 (m, 2H), 3.80 (br, 1H), 4.43 (br, 1H), 4.95 (br, 1H), 5.25 (d, 1H, J=3.8 Hz), 6.11 (m, 1H), 6.47 (br, 1H), 7.90 (s, 1H).

### (Example 2)

8,2'-Anhydro-8-mercapto-9-β-arabinofuranosylguanine (100 mg, 0.34 mmol), water (5.0 mL), 2M aqueous sodium hydroxide solution (0.34 mL) and Raney-nickel (500 mg, Kawaken Fine Chemicals Co., Ltd., NDHT-M0) were added to a pear-shaped flask (50 mL) and the reaction mixture (pH 13.1) was stirred at 95°C for 15 hr. The reaction mixture was filtered through Kiriyama funnel under reduced pressure. The obtained solution was analyzed by HPLC to confirm that 2'-deoxyguanosine was obtained in the yield of 24% (0.08 mmol). It was also confirmed that 8,2'-anhydro-8-mercapto-9-β-arabinofuranosylguanine (0.15 mmol, 45%), a starting material, was recovered.

### (Example 3)

A Ni-Al alloy powder (10 g, Kawaken Fine Chemicals Co., Ltd., NDH) was added to a two-necked pear shaped flask (200 mL), and the inside of the flask was purged with argon gas. 8,2'-Anhydro-8-mercapto-9-β-arabinofuranosylguanine (5 g, 16.8 mmol) was dissolved in 2M aqueous sodium hydroxide solution (110 mL), and the solution (pH 14.3) was slowly added dropwise to the reaction system. The reaction mixture was stirred at 60°C for 15 hr. The mixture was filtered through a Kiriyama funnel under reduced pressure and washed with water (35 mL). The obtained solution was analyzed by HPLC to confirm that 2'-deoxyguanosine was obtained in the yield of 53% (8.93 mmol).

### (Example 4)

A Ni-Al alloy powder (200 mg, Kawaken Fine Chemicals Co., Ltd., NDH) was added to a two-necked pear shaped flask (50 mL), and the inside of the flask was purged with argon gas. 8,2'-Anhydro-8-mercapto-9-β-arabinofuranosylguanine (100 mg, 0.34 mmol) was dissolved in 2M aqueous sodium hydroxide solution (2.2 mL), and the solution (pH 14.3) was slowly added dropwise to the reaction system. The reaction mixture was stirred at 60°C for 4 hr. The mixture was filtered through a Kiriyama funnel under reduced pressure and washed with water (20 mL). The obtained solution was analyzed by HPLC to confirm that 2'-deoxyguanosine was obtained in the yield of 44% (0.15 mmol). It was also confirmed that 8,2'-anhydro-8-mercapto-9-β-arabinofuranosylguanine (0.02 mmol, 5%), a starting material, was recovered.

### (2) Conversion of 2'-deoxyguanosine to N²,3',5'-triisobutyryl-2'-deoxyguanosine

### (Example 5)

An aqueous sodium hydroxide solution containing 2'-deoxyguanosine (443.6 mg, 1.66 mmol) obtained in Example 1 was added to a wide-mouthed pear shaped flask (100 mL). The mixture was adjusted to pH 7 using 2M hydrochloric acid at 0°C. The obtained white suspension was concentrated under reduced pressure by a rotary evaporator and azeotropic operation with toluene (5 mL) was performed twice. Furthermore, azeotropic operation with pyridine (5 mL) was performed twice. Pyridine (8 mL) and chloroform (12 mL) were added and the mixture was cooled to 0°C. Isobutyryl chloride (1.8 mL, 16.8 mmol) was added and the mixture was stirred at 0°C for 30 min. and at room temperature for 15 hr. Water was added to the reaction system to quench the reaction. The reaction mixture was extracted 3 times with chloroform (40 mL). The organic layer was washed with 5% aqueous sodium hydrogen carbonate solution and further washed with water. The organic layer was filtered through Kiriyama funnel to remove a white solid. The obtained organic layer was dried over magnesium sulfate and magnesium sulfate was filtered through Kiriyama funnel. The organic layer was concentrated under reduced pressure and the residue was purified by column chromatography. The fraction was concentrated under reduced pressure to give N²,3',5'-triisobutyryl-2'-deoxyguanosine (517.6 mg, 1.08 mmol, 65% (2'-deoxyguanosine standard)).

### Industrial Applicability

According to the present invention, 2'-deoxyguanosine compound can be obtained in a high yield from an 8,2'-anhydro-8-mercapto-9-β-arabinofuranosylguanine compound. Moreover, the amount of nickel reagent can be markedly reduced by carrying out the reaction using nickel-aluminum alloy, as compared to the use of nickel such as Raney-nickel etc. In addition, a 2'-deoxyguanosine compound can be produced safely, because preparation of highly active Raney-nickel prior to the desulfurization reaction becomes unnecessary.

Although only some exemplary embodiments of this invention have been described in detail above, those skilled in the art will readily appreciated that many modifications are possible in the exemplary embodiments without materially departing from the novel teachings and advantages of this invention. Accordingly, all such modifications are intended to be included within the scope of this invention.

## Claims

1. A method of producing a 2'-deoxyguanosine compound represented by the following formula (2) 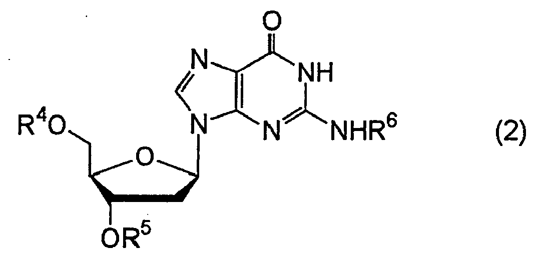 wherein R⁴ and R⁵ are each independently a hydrogen atom or a hydroxyl-protecting group and R⁶ is a hydrogen atom or an amino-protecting group, which comprises desulfurizing an 8,2'-anhydro-8-mercapto-9-β-arabinofuranosylguanine compound represented by the following formula (1) 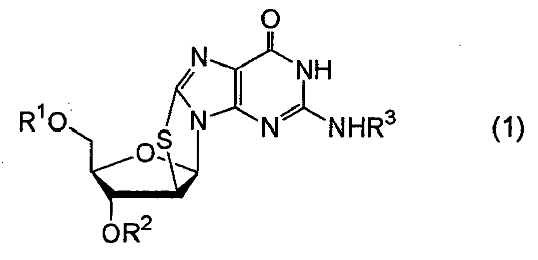 wherein R¹ and R² are each independently a hydrogen atom or a hydroxyl-protecting group and R³ is a hydrogen atom or an amino-protecting group, in the presence of nickel or a nickel alloy, a solvent, and a base.

2. A method of producing a 2'-deoxyguanosine compound represented by the following formula (3) 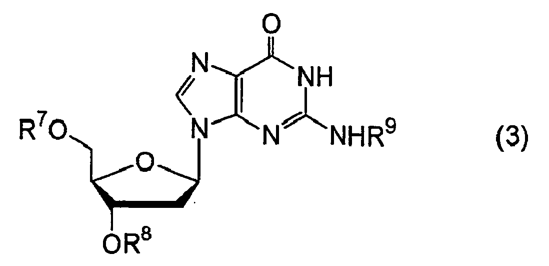 wherein R⁷ and R⁸ are each independently a hydrogen atom or a hydroxyl-protecting group, R⁹ is a hydrogen atom or an amino-protecting group, provided that R⁷, R⁸ and R⁹ are not simultaneously hydrogen atoms, which comprises obtaining a 2'-deoxyguanosine compound represented by the formula (2) according to the production method of claim 1, and when one or more of R⁴, R⁵ and R⁶ of the compound are hydrogen atoms, replacing one or more of the hydrogen atoms with protecting group(s).

3. The production method of claim 1, wherein R¹, R², R³, R⁴, R⁵ and R⁶ are hydrogen atoms.

4. The production method of claim 3, which further comprises a step of replacing hydrogen atoms for R⁴, R⁵ and R⁶ with protecting groups.

5. The production method of any of claims 1 to 4, wherein the solvent is water.

6. The production method of any of claims 1 to 5, wherein the desulfurization is carried out in a liquid which comprises a base and a solvent and which has a pH of not less than 11.

7. The production method of any of claims 1 to 6, wherein the nickel or nickel alloy is added in an amount of 50-1500 mass% of the total mass of the 8,2'-anhydro-8-mercapto-9-β-arabinofuranosylguanine compound.

8. The production method of any of claims 1 to 7, wherein the desulfurization reaction is carried out at a temperature of 40-100°C.

9. The production method of any of claims 1 to 8, wherein the nickel alloy is a nickel-aluminum alloy.
